# EUROPEAN PATENT SPECIFICATION

(11) **EP 2 682 071 B1**
(45) Date of publication and mention of the grant of the patent: **22.04.2015**
(21) Application number: 13174586.1
(22) Date of filing: 01.07.2013
(51) Int. Cl.: A61C 13/00, A61C 9/00

(54) **Method for copying and reproducing anatomical segments of a patient**
Verfahren zum Kopieren und Nachbilden von anatomischen Segmenten eines Patienten
Procédé permettant de copier et de reproduire des segments anatomiques d'un patient

(30) Priority: 02.07.2012 IT BO20120362
(43) Date of publication of application: 08.01.2014
(73) Proprietor: Villa Elisa Soverato S.r.l., 88068 Soverato ( (CZ) (IT)
(72) Inventor: Froio, Bruno, 88068 Soverato (CZ) (IT); Froio, Salvatore, 88068 Soverato (CZ) (IT); Wagerle, Giovanni Christian, 88068 Soverato (CZ) (IT); Toselli, Roberto, 40121 Bologna (IT)
(74) Representative: Negrini, Elena

(56) References cited:
- WO-A1-2011/077175
- WO-A2-2007/062658

## Description

The present invention relates to the fields concerning the realization of anatomic prostheses for humans and animals and the development of operating procedures and in particular relates to a method for copying and reproducing anatomical segment of patients.

At present the techniques for copying anatomical segments requires using cast of the part in order to obtain a negative of the same which forms a mould that later will be used to produce the copy of the part using common casting techniques.

It is known that, for example, in the dental field the copy of the dental arch is realized by means of cast obtained using various types of silicones and/or alginates. To realize fixed prostheses, sometimes, the cast is realized after the patient has been locally anesthetized to limit both unwanted movement and the annoyance due to the operations.

Such materials are kept in position for a time sufficient to guarantee the negative reproduction of the dental arch, so that they can be handled without risk of deforming and later they are removed from the patient's mouth.

Once the mixture has sufficiently hardened, showing the negative form of the affected portion, the operator prepares the mold for the subsequent casting of the resin used to obtain the positive copy of the dental arch.

A disadvantage of said known copy methods of anatomic segments regards the high risk of errors in the phase of anatomical portion mould preparation due to the possibility of movement of the tissues, especially the soft ones, when they are solicited during the mould preparation, and the number of operations to be performed before getting the finished product.

Further disadvantage of known methods regards their invasiveness on the patient during the evaluation step wherein the patient must remain, even for a long time, in uncomfortable positions and in contact with chemicals that are not always well tolerated.

Each document WO 2007/062658 and WO 2011/077175 discloses a method for copying and reproducing anatomical segments of patients comprising the steps of:
- taking a three-dimensional acquisition of the anatomical segment to be reproduced;
- digitally saving the three-dimensional acquisition data;
- sending the digital data to a three-dimensional printing device suitable to make a copy of the anatomical segment.

One object of the present invention is to propose a method for copying anatomical segments in an automated way that improves the precision of copying anatomical segments and that reduces the possibilities of error in the step of acquisition of the concerned anatomical part.

Further object is to propose a method that eliminates the use of substances being hazardous for the operator, such as quartz, asbestos, vapors of methyl methalcrylate which may remain in the work environment also for several hours, and a method which is not invasive for the patient as it doesn't require traditional cast preparation.

Another object is to propose a method that allows to reduce the anatomical implants production time and to improve the accuracy of reproduction of the same.

Further object is to propose a method for reproduce anatomical segments that allows developing in a rapid and economic way specific operating strategies for each intervention, reproducing the anatomical portion concerned and simulating on it the intervention.

These objects are fulfilled by the subject-matter of claim 1.

In the dental field, three-dimensional acquisition of anatomical segments is performed using diagnostic techniques that involve high-definition digital orthopantomographs. These orthopantomographs allow solving the anatomical details of the size of the order of 70 microns or less.

To realize the three-dimensional acquisition of the other anatomical segments it is preferable to use high-definition computed tomography devices or three-dimensional X-ray devices.

The above mentioned examinations allow acquiring and subsequently displaying of X-ray data of patient information such as solid volume assemblies.

During the three-dimensional acquisition or during the subsequent acquired raw data processing the different tissues constituting the acquired anatomical segment are evidenced using different colors, such as bone, muscle, tendons, blood vessels, teeth, nerve endings, and other tissues, thereby enabling a rapid and precise identification of the same in the subsequent steps.

The, raw or processed, data obtained by means of the three-dimensional acquisition are saved in a digital format according "Dicom" or "Stl" standard to be easily read and interpreted by different computer programs, and to be sent to a three-dimensional printing device.

The three-dimensional printing of the anatomic segment copy is performed by means of additive stratification devices or of "rapid prototyping" performing a deposition of successive layers of polymeric material based on silicone according to the shape of the anatomical segment.

The polymeric material is colored according to the tissue to be reproduced.

Moreover, to reproduce the specific density of different tissues, the three-dimensional printing is locally done by varying the density of the deposited material according to the particular tissue. The material used is one of polymeric type silicone based.

As an alternative to additive stratification devices it can be used a stereo-lithography device.

After the three-dimensional printing the obtained object is a polychromatic copy in 1:1 scale of the interested segment anatomical on which different tissues are highlighted.

The so obtained copy is suitable to be used as a model to develop operating strategies on that anatomical segment exploiting the polychromatic representation of the copy to easily identify the different tissues and any parts to be removed, or on which the patient should be operated.

Similarly, such copy can be used to perform virtual autopsies and forensic investigations without having to intervene directly on the body damaging it.

The so obtained copy also allows designing and making prosthesis for each specific patient and in this case there is a variant of the preferred embodiment of the method that after the three-dimensional printing provides:
- acquiring a copy of the anatomical segment;
- generating a solid model from the acquisition data of the copy;
- designing the prosthesis based on the obtained solid model obtained;
- making said prosthesis.

The digital acquisition of the anatomical segment copy occurs by means of laser scanner mounted on multiple axis manipulators to vary the mutual position between the acquisition device and copy. This position variation allows acquiring also not directly visible areas of the copy, such as inside grooves or recesses.

As an alternative to laser scanners it can be used a mechanical probe attached to an articulated arm and sliding on the copy surface copy and reading out the coordinates.

The three-dimensional acquisition information of the copy are saved in a digital format compatible with computer assisted designing (CAD) or computer-aided manufacturing (CAM) programs and represent digital solid modes of the anatomical segment copy.

Based on the so obtained digital solid model a digital model of prosthesis to be implanted in the patient's anatomical segment is designed and developed.

The prosthesis realization is achieved by means of numerical control cutting machines.

The method allows to reproduce in a non-invasive, three-dimensional, polychromatic with variable densities way different anatomical segments of human body such as: skeleton, joints, teeth, brain, neck, chest, heart, lungs, arterial and venous vascular system, abdomen, liver , pancreas, spleen, digestive tract, uterus, kidneys, adrenal glands, urethra, bladder and prostate.

Advantageously, the method above described allows the complete automation of the operations necessary to make a copy of one or more anatomical segments of a patient allowing a considerable saving of time compared to known techniques.

Furthermore the above described method greatly reduces the stress which is subject the patient as it eliminates the need to perform a mold of anatomical segment to reproduce and simultaneously allows an improvement of the quality and accuracy in the acquisition step as the patient is in a relaxed condition and is not subject to any load or stress.

Finally, the above described method allows reproducing any type of tissue by means of material having a chromatic diversification. In such a way operators can achieve a more careful preparation of the intervention or can develop specific prosthesis for every problem having the opportunity to test them in advance on a copy of the anatomical segment.

Furthermore, the above described method allows the reconstruction of a copy of human body parts by means of not invasive and/or not destructive acquisition methods allowing the execution of post-mortem or forensic examinations even in case of not repeatability.

An advantage of the present invention is to provide a method for copying anatomical segments in an automated way improving the copying anatomical segments precision and reducing the error possibilities in the acquisition step of concerned anatomical part.

Further advantage is to provide a method that eliminates the use of hazardous substances for the operator and that is not invasive for the patient because it does not require a mould preparation.

Other advantage is to provide a method that allows to reduce the anatomical implants production time and that improves the accuracy of realizing the same.

Further advantage is to provide a method for reproducing anatomical segments that allows developing in a rapid and economic way specific operating strategies for every intervention reproducing the anatomical portion concerned and simulating on it the intervention.

## Claims

1. Method for copying and reproducing anatomical segments of a patient comprising the following steps:
- taking a three-dimensional acquisition of the anatomical segment to be reproduced;
- digitally saving the data of the three-dimensional acquisition;
- sending such digital data to a three-dimensional printing device suitable to reproduce a copy of the anatomical segment;
said method being **characterized by**:
- identifying in the digital data the volumes corresponding to the different anatomical tissue that form the acquired anatomical segment and identifying them with different colors or properties;
- deposing in layer sequence some polymeric material colored according to the tissue to be copied;
- using silicon based polymeric material having a density comparable with the density of the tissue to be copied.

2. Method according to claim 1 **characterized by** taking the three-dimensional acquisition by means of digital orthopantomographs or volumetric radiography or computerized tomography.

3. Method according to any previous claims **characterized by** the fact of making the copy by means of additive stratification devices or stereo lithography.

4. Method according to any previous claims **characterized by** saving the data in a digital format according to "Dicom" or "Stl" standards.

5. Method according to any previous claims **characterized by:**
- digital acquiring, by means of a three-dimensional laser scanner or a mechanical feeler, the so obtained anatomical segment copy;
- saving a model containing the information of the digitized copy;
- elaborating the model by CAD or CAM design programs.

6. Method according to claim 5 **characterized by**:
- elaborating a digital model of a prosthesis to be inserted in the patient anatomical segment;
- making the prosthesis by computer numerical controlled cutting machine.

## Patentansprüche

1. Verfahren zum Kopieren und Nachbilden von anatomischen Segmenten eines Patienten, umfassend die folgenden Schritte:
- Aufnehmen einer dreidimensionalen Erfassung des anatomischen Segments, das nachgebildet werden soll;
- digitales Speichern der Daten der dreidimensionalen Erfassung;
- Senden dieser digitalen Daten an eine 3D-Druckvorrichtung (3D = dreidimensional), die geeignet ist, eine Kopie des anatomischen Segments nachzubilden;
und das Verfahren ist **gekennzeichnet durch** die Schritte:
- Identifizieren der Volumina innerhalb der digitalen Daten, die dem unterschiedlichen anatomischen Gewebe entsprechen, das das erfasste anatomische Segment bildet, und Identifizieren dieser mit verschiedenen Farben oder Eigenschaften;
- Aufbringen von einem polymeren Material in einer Schichtfolge, das entsprechend dem zu kopierenden Gewebe gefärbt ist; und
- Verwenden von Silikon-basiertem polymerem Material, das eine Dichte aufweist, die mit der Dichte des zu kopierenden Gewebes vergleichbar ist.

2. Verfahren gemäß Patentanspruch 1, **gekennzeichnet durch** das Aufnehmen der dreidimensionalen Erfassung mithilfe von digitalen Orthopantomographen oder volumetrischer Radiographie oder Computertomographie.

3. Verfahren gemäß einem der vorhergehenden Patentansprüche, **gekennzeichnet durch** das Anfertigen der Kopie mithilfe von zusätzlichen Aufschichtungsvorrichtungen oder von Stereolithographie.

4. Verfahren gemäß einem der vorhergehenden Patentansprüche, **gekennzeichnet durch** das Speichern der Daten in einem digitalen Format gemäß "Dicom"- oder "Stl"-Standard.

5. Verfahren gemäß einem der vorhergehenden Patentansprüche, **gekennzeichnet durch** die weiteren Schritte:
- digitales Erfassen der so erhaltenen Kopie des anatomischen Segments mithilfe eines 3D-Laser-Scanners oder eines mechanischen Fühlers (3D = dreidimensional);
- Speichern eines Modells, das die Infomationen der digitalisierten Kopie beinhaltet; und
- Ausarbeiten des Modells mithilfe von CAD- oder CAM-Design-Programmen.

6. Verfahren gemäß Patentanspruch 5, **gekennzeichnet durch** die weiteren Schritte:
- Ausarbeiten eines digitalen Modells einer Prothese, die in das anatomische Segment des Patienten eingesetzt werden soll; und
- Herstellen der Prothese mithilfe einer CNC-Schneidemaschine (Schneidmaschine mit computerisierter numerischer Steuerung).

## Revendications

1. Procédé destiné à copier et à reproduire des segments anatomiques d'un patient, comprenant les étapes suivantes consistant à :
- procéder à une acquisition tridimensionnelle du segment anatomique à reproduire ;
- sauvegarder de manière numérique les données de l'acquisition tridimensionnelle ;
- envoyer de telles données numériques à un dispositif d'impression tridimensionnel approprié à la reproduction d'une copie du segment anatomique ;
ledit procédé étant **caractérisé par** les étapes consistant à :
- identifier, dans les données numériques, les volumes qui correspondent au tissu anatomique différent, qui forment le segment anatomique acquis et les identifier par des couleurs ou des propriétés différentes ;
- déposer en couches successives un certain matériau polymère coloré selon le tissu à copier ;
- utiliser un matériau polymère à base de silicone qui présente une densité comparable à la densité du tissu à copier.

2. Procédé selon la revendication 1, **caractérisé par** une étape consistant à procéder à l'acquisition tridimensionnelle au moyen d'une orthopantomographie numérique, d'une radiographie volumétrique ou d'une tomodensitométrie.

3. Procédé selon l'une quelconque des revendications précédentes, **caractérisé par le fait qu'**il comprend une étape consistant à réaliser la copie au moyen de dispositifs de stratification additive ou de stéréolithographie.

4. Procédé selon l'une quelconque des revendications précédentes, **caractérisé par le fait qu'**il comprend une étape consistant à sauvegarder les données dans un format numérique selon les normes « Dicom » ou « Sti ».

5. Procédé selon l'une quelconque des revendications précédentes, **caractérisé par** les étapes consistant à :
- acquérir de manière numérique, au moyen d'un dispositif de balayage à laser tridimensionnel ou d'un palpeur mécanique, la copie de segment anatomique ainsi obtenue ;
- sauvegarder un modèle qui contient les informations de la copie numérisée ;
- élaborer le modèle à l'aide de programmes de conception de CAO ou de FAO.

6. Procédé selon la revendication 5, **caractérisé par** les étapes consistant à :
- élaborer un modèle numérique d'une prothèse à insérer dans le segment anatomique d'un patient ;
- réaliser la prothèse à l'aide d'une machine de découpe à commande numérique par ordinateur.
